# EUROPEAN PATENT APPLICATION

(11) **EP 4 553 844 A1**
(43) Date of publication of application: **14.05.2025**
(21) Application number: 24207155.3
(22) Date of filing: 17.10.2024
(51) Int. Cl.: G16H 20/40, G16H 50/20, G16H 50/30, G16H 50/70, G16H 80/00, A61N 5/00

(54) **USING LARGE LANGUAGE MODELS TO TEST THE VALIDITY OF A USER ACTION IN TREATMENT PLANNING**

(30) Priority: 07.11.2023 US 202318387794
(71) Applicant: Siemens Healthineers International AG, 6312 Steinhausen (CH)
(72) Inventor: KUUSELA, Esa, 02320 Espoo (FI); HAUTALA, Ismo, 02600 Espoo (FI)
(74) Representative: Mathisen & Macara LLP

(57) **Abstract**

Disclosed herein are methods (200) and systems (100) to evaluate cost values for different radiotherapy treatment plans using external AI models (111). A method comprises presenting (202) a user interface (300) providing an interaction interface between a plurality of medical professionals communicating regarding a radiation therapy treatment of a patient during a tumor board meeting or a radiotherapy treatment planning process; receiving (204), from the interaction interface, a first input comprising a first patient attribute of the patient and a second input corresponding to the radiation therapy treatment of the patient; executing (206) a machine learning language processing model using the first input and the second input to predict a task associated with generating a treatment plan for the patient; receiving (208), from the interaction interface, a third input; and when (210) the third input does not correspond to the predicted task, presenting an indication of the predicted task.

## Description

### TECHNICAL FIELD

This application relates generally to generating a radiotherapy treatment plan using a machine learning language processing model.

### BACKGROUND

Radiotherapy or radiation therapy (RT) is one of the main modalities used in cancer treatment, and RT treatment planning (RTTP) is a complex process that contains specific guidelines, protocols, and instructions adopted by different medical professionals, such as clinicians, medical device manufacturers, and the like. Typically, the overall treatment strategy, such as selecting which RT modalities should be used, is discussed and decided in a multi-disciplinary tumor board meeting, where one or more RT-oncologists are accompanied by surgeons, radiologists, and general physicians, among others. These meetings should thus be as efficient as possible since multiple key resources of the clinic are participating in the tumor board's reviews and decisions.

RTTP creation typically involves a collaboration of multiple professionals who collectively function as a tumor board who diagnose patients and develop the RTTP for a patient. Especially important in this process is the interaction between the members of the tumor board and providers (e.g., radiation oncologists). For an example, an oncologist may first generate a plan for treatment using a computer model. Members of the tumor board may discuss a treatment plan generated by the oncologist during a tumor board review. Such discussions may be memorialized as the RTTP or tasks of the RTTP. The discussions by the tumor board during meetings often contain useful information that could be cross-referenced and verified to further develop RTTP options or tasks, though this information is rarely captured and analyzed in a manner that could benefit downstream computing processes or medical tasks.

### SUMMARY

In accordance with a first aspect of the invention, there is provided a method as defined by claim 1.

In accordance with a second aspect of the invention, there is provided a system as defined by claim 8.

Optional features are defined by the dependent claims.

Radiation therapy (RT) treatment planning can require a chain of multiple actions performed by several professionals in different roles. Typically, a high-quality error-free planning process needs synchronized actions and checks from multiple personnel roles. This requires a significant amount of effort and unnecessary waiting which can result in delayed treatment.

Systems that have tried to overcome technical plan quality control issues have involved checking dosimetrical plan quality and comparing the dosimetrical plan quality to historical data. For example, a computer can compare organ Dose-Volume-Histogram (DVH) curves to predict organ dose volumes based on existing and/or historical plans. Similar approaches may be used for three-dimensional dosage prediction or predicting whether certain clinical goals should be obtained. In another example, a computer may manually delineate organs and validate the delineation using an auto-segmentation model (e.g., a machine learning model that is trained using historical, curated delineated organs). However, these approaches have technical shortcomings such as the approaches may be limited to identifying deviations from a dosage distribution or other pre-defined numerical data, such as organ masks. There are a number of other types of errors that may occur as well, such as the approaches may result in selecting a wrong treatment template, omitting special information related to the patient, or generating sub-optimal solutions in general without having an impact on the observed quantities that would be strong enough to be noticed by users.

A computer implementing the systems and methods described herein may overcome the aforementioned technical deficiencies using a machine learning language processing model (e.g., a large language model (LLM)). For example, a computer can use a machine learning language processing model to perform checks to the validity of the planning process by trying to predict the next tokens in a user feed based on previous information (e.g., patient attributes and/or treatment attributes) related to a patient for which an RTTP is being generated. The computer can perform the prediction using a specially trained machine learning language processing model using 'zero-shot' prompting or a generic machine learning language processing model using 'single-shot' or 'few-shot' prompting. The computer can compare predicted tasks or treatment attributes for the patient with an input task or treatment attribute of a patient input by a user (e.g., an oncologist or a member of a tumor board during a tumor board review or meeting, or a dosimetrist or a physicist during the course of the RTTP process). If the prediction differs from the input task or treatment attribute, the computer can prompt the machine learning language processing model to output a message indicating the difference. In some cases, the machine learning language processing model may include a rationale or reasoning regarding the predicted treatment attribute or decision. A decision can be the same as a task for treating a patient. The user can view the rationale and the message and provide an input indicating whether to accept the machine learning language processing model's predicted decision or to reject the machine learning language processing model's proposal.

For example, human users such as a tumor board, in some cases including one or more oncologists, may review a patient's case to determine treatment for the patient during a tumor board meeting or radiotherapy treatment planning process. In doing so, the members of the tumor board or participants in the radiotherapy treatment process may communicate with each other by holding a conversation. The members or participants may hold the conversation via an electronic platform hosted by a computer. The electronic platform may comprise MS Teams^{®}, Skype^{®}, WebEx^{®}, Slack^{®}, and Twilio^{®}, among others. The members or participants may type in inputs into the electronic platform to hold a conversation with each other similar to an instant messaging application. The computer may store the inputs or process the inputs using language processing techniques to extract specific types of data, such as treatment attributes or patient attributes of the patient. In some cases, the members or participants may hold an in-person meeting or discussion. In such cases, the members or participants may be recorded by a recording device and the recording device may transmit the record to the computer hosting the electronic platform. The computing device may transcribe the recording into a transcript and similarly store the transcript, or patient attributes or treatment attributes from the transcript, in memory. The computer may additionally store previously input patient attributes and/or treatment attributes for the patient in memory.

The computer can monitor the conversation by the human users, e.g., by the tumor board, over the course of the human user meeting or the tumor board meeting. The computer can process the text inputs or transcriptions of the conversation to determine when the members or participants have made a decision regarding treatment or a next stage in determining treatment. The computer can detect the decision responsive to detecting an end to the tumor board meeting or the RTTP planning discussion and/or responsive to detecting the decision in the middle of the tumor board meeting or the RTTP planning discussion using natural language processing techniques. Responsive to detecting the decision, the computer can use the inputs collected over the course of the tumor board meeting or the RTTP planning discussion and/or any patient or treatment attributes stored in memory for the patient being evaluated to determine a predicted decision. The computer can do so by generating a prompt from the collected data and executing the machine learning language processing model using the prompt as input. Based on the execution, the machine learning language processing model can output a predicted decision. The computer can compare the decision detected from the inputs to the predicted decision to determine if the predicted decision and input decision differ (e.g., are not an exact match or differ by an amount above a threshold). Responsive to determining the predicted decision does not differ from the input decision, the computer can execute the machine learning language processing model to generate a message indicating the input decision is correct and display the message at one or more computing devices of the board members or participants attending the tumor board meeting or the RTTP planning discussion.

However, responsive to determining the predicted decision and input decision differ, the computer can generate a message indicating the difference and display the message to one or more of the board members or the RTTP planning discussion participants. For example, the computer can prompt the machine learning language processing model to generate a message indicating there is a difference. In some cases, the computer can prompt the machine learning language processing model to generate the message with an indication of any patient or treatment attributes that may have caused the difference. The machine learning language processing model may generate the message accordingly and the computer can display the message to the board members. In some cases, the computer can train the machine learning language processing model based on the difference.

Accordingly, the computer implementing the systems and methods described herein can use a machine learning language processing model to improve RTTP in real time, avoiding iterative execution approaches to determining the best forms of treatment. The computer can improve the system over time by continually evaluating the performance of the model compared to implemented decisions for treatment, and training the machine learning language processing model.

In an embodiment, a method includes presenting, by a processor, a user interface providing an interaction interface between a plurality of human users or medical professionals communicating regarding a radiation therapy treatment of a patient during a tumor board meeting or a radiotherapy treatment planning process; receiving, by the processor from the interaction interface, a first input comprising a first patient attribute of the patient and a second input corresponding to the radiation therapy treatment of the patient; executing, by the processor, a machine learning language processing model using the first input and the second input to predict a task associated with generating a treatment plan for the patient, wherein the machine learning language processing model is trained using a set of transcriptions of a set of tumor board meetings or radiotherapy treatment planning processes (e.g., transcriptions of a set of instructions given by a radiotherapy treatment oncologist to one or more radiotherapy treatment experts) for a set of previously implemented radiation therapy treatments and a hierarchy of tasks associated with generating treatment plans; receiving, by the processor from the interaction interface, a third input; and when the third input does not correspond to the predicted task presenting, by the processor on the interaction interface, an indication of the predicted task.

The method may include, when the third input corresponds with the predicted task executing, by the processor, an analytical protocol to identify a value for the predicted task; and presenting, by the processor, the value for the predicted task on the interaction interface.

The predicted task may be associated with a timeline of radiation therapy treatment of the patient.

The method may further comprise transmitting, by the processor, the first input, the second input, and a response to the predicted task to a radiation therapy plan optimizer as an instruction to generate a radiotherapy treatment plan for the patient.

The method may further comprise presenting, by the processor on the interaction interface, a hyperlink configured to direct the interaction interface to third-party data associated with the predicted task.

The method may further comprise receiving, by the processor, a response to presentation of the predicted task; and retraining, by the processor, the machine learning language processing model using the response.

The method may further comprise generating, by the processor, an alternative workflow that does not include the predicted task.

In another embodiment, a system includes a server comprising a processor and a non-transitory computer-readable medium containing instructions that when executed by the processor causes the processor to present a user interface providing an interaction interface between a plurality of human users or medical professionals communicating regarding a radiation therapy treatment of a patient during a tumor board meeting or a radiotherapy treatment planning process; receive, from the interaction interface, a first input comprising a first patient attribute of the patient and a second input corresponding to the radiation therapy treatment of the patient; execute a machine learning language processing model using the first input and the second input to predict a task associated with generating a treatment plan for the patient, wherein the machine learning language processing model is trained using a set of transcriptions of a set of tumor board meetings or radiotherapy treatment planning processes (e.g., transcriptions of a set of instructions given by a radiotherapy treatment oncologist to one or more radiotherapy treatment experts) for a set of previously implemented radiation therapy treatments and a hierarchy of tasks associated with generating treatment plans; receive, from the interaction interface, a third input; and when the third input does not correspond to with the predicted task, present, on the interaction interface, an indication of the predicted task.

The instructions may further cause the processor to: when the third input corresponds with the predicted task, execute an analytical protocol to identify a value for the predicted task; and present the value for the predicted task on the interaction interface.

The predicted task may be associated with a timeline of radiation therapy treatment of the patient.

The instructions may further cause the processor to transmit the first input, the second input, and a response to the predicted task to a radiation therapy plan optimizer as an instruction to generate a radiotherapy treatment plan for the patient.

The instructions may further cause the processor to present, on the interaction interface, a hyperlink configured to direct the interaction interface to third-party data associated with the predicted task.

The instructions may further cause the processor to receive a response to presentation of the predicted task; and retrain the machine learning language processing model using the response.

The instructions may further cause the processor to generate an alternative workflow that does not include the predicted task.

In yet another embodiment, a system includes a computer configured to display a user interface; and a server in communication with the computer. The server can be configured to present the user interface providing an interaction interface between a plurality of human users or medical professionals communicating regarding a radiation therapy treatment of a patient during a tumor board meeting or a radiotherapy treatment planning process; receive, from the interaction interface, a first input comprising a first patient attribute of the patient and a second input corresponding to the radiation therapy treatment of the patient; execute a machine learning language processing model using the first input and the second input to predict a task associated with generating a treatment plan for the patient, wherein the machine learning language processing model is trained using a set of transcriptions of a set of tumor board meetings or radiotherapy treatment planning processes (e.g., transcriptions of a set of instructions given by a radiotherapy treatment oncologist to one or more radiotherapy treatment experts) for a set of previously implemented radiation therapy treatments and a hierarchy of tasks associated with generating treatment plans; receive, from the interaction interface, a third input; and when the third input does not correspond to the predicted task, present, on the interaction interface, an indication of the predicted task.

The server may be further configured to when the third input corresponds to the predicted task execute an analytical protocol to identify a value for the predicted task; and present the value for the predicted task on the interaction interface.

The predicted task may be associated with a timeline of radiation therapy treatment of the patient.

The server may be further configured to transmit the first input, the second input, and a response to the predicted task to a radiation therapy plan optimizer as an instruction to generate a radiotherapy treatment plan for the patient.

The server may be further configured to present, on the interaction interface, a hyperlink configured to direct the interaction interface to third-party data associated with the predicted task.

The server may be further configured to receive a response to presentation of the predicted task; and retrain the machine learning language processing model using the response.

The server may be further configured to generate an alternative workflow that does not include the predicted task.

In an example, the machine learning language processing model may be trained to contextually continue a conversation with the tumor board or participants in the RTTP process, based upon the text, or transcribed text, of the medical discussion and patient-related data indicating patient attributes over time.

### BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.
**FIG. 1** illustrates components of a system for conducting human user discussions, tumor board discussions and/or RTTP planning, and developing RTTPs using a machine learning language processing model, according to an embodiment.
**FIG. 2** shows an operational workflow of a method performed in hardware and software computing components that host and execute a machine learning language processing model, in accordance with an embodiment.
**FIG. 3** depicts an example user interface of collaboration software for member-users of a tumor board discussion or the participants in an RTTP planning process to collaborate and interact with a machine learning language processing model, according to an embodiment.
**FIG. 4** illustrates a process flow diagram executed in a machine learning language processing plan generation system, according to an embodiment.
**FIG. 5** illustrates a dataflow amongst components of a system for hosting human user, tumor board or RTTP planning process collaboration and radiotherapy treatment plan generation, according to an embodiment.

### DETAILED DESCRIPTION

Reference will now be made to the illustrative embodiments depicted in the drawings, and specific language will be used here to describe the same. It will nevertheless be understood that no limitation of the scope of the claims or this disclosure is thereby intended. Alterations and further modifications of the inventive features illustrated herein, and additional applications of the principles of the subject matter illustrated herein, which would occur to one skilled in the relevant art and having possession of this disclosure, are to be considered within the scope of the subject matter disclosed herein. Other embodiments may be used and/or other changes may be made without departing from the scope of the present disclosure. The illustrative embodiments described in the detailed description are not meant to be limiting of the subject matter presented.

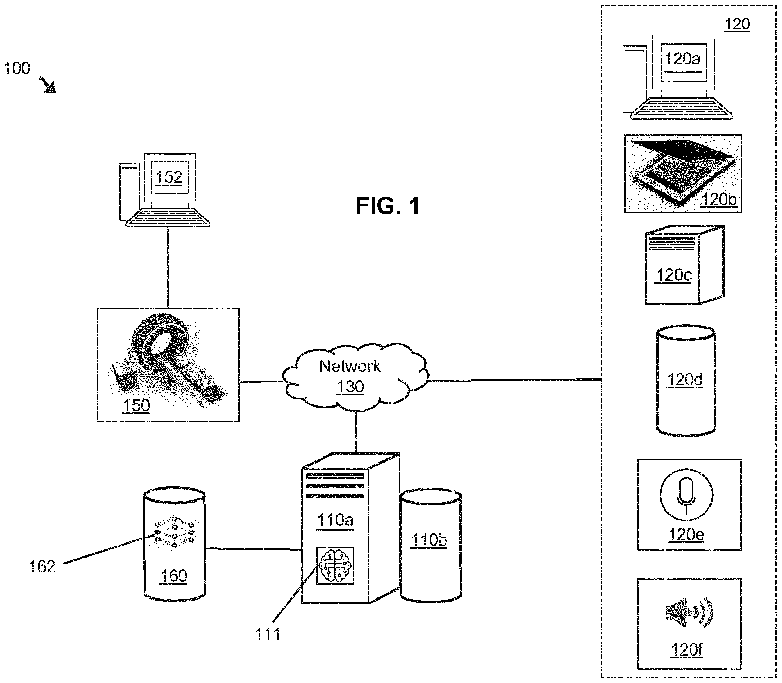

**FIG. 1** illustrates components of a system **100** for conducting human user discussions, tumor board discussions or steps of the RTTP process in order to develop RTTPs using a machine learning language processing model, according to an embodiment. The system **100** may include an analytics server **110a**, a system database **110b,** a machine learning language processing model **111** for presenting contextual information to participants of a medical discussion, end-user devices **120a-120f** (collectively end-user devices **120**), a medical device **150,** a medical device computer **152**, a database **160**, and a radiotherapy plan optimizer **162.** Various components depicted in **FIG. 1** may belong to a radiation therapy treatment clinic at which patients may receive radiation therapy treatment, in some cases via one or more radiation therapy machines (e.g., the medical device **150**).

The system **100** is not confined to the components described herein and may include additional or other components, not shown for brevity, which are to be considered within the scope of the embodiments described herein.

The above-mentioned components may be connected to each other through one or more networks **130**. Examples of the network **130** may include, but are not limited to, private or public local-area networks (LAN), wireless local-area networks (WLAN), metropolitan-area networks (MAN), wide-area networks (WAN), and the Internet. The network **130** may include wired and/or wireless communications according to one or more standards and/or via one or more transport mediums. The communication over the network **130** may be performed in accordance with various communication protocols such as Transmission Control Protocol and Internet Protocol (TCP/IP), User Datagram Protocol (UDP), and IEEE communication protocols. In one example, the network **130** may include wireless communications according to Bluetooth specification sets or another standard or proprietary wireless communication protocol. In another example, the network **130** may also include communications over a cellular network, including, e.g., a GSM (Global System for Mobile Communications), CDMA (Code Division Multiple Access), or EDGE (Enhanced Data for Global Evolution) network.

The analytics server **110a** may generate and display an electronic platform configured to interface a user with the machine learning language processing model **111** and for receiving patient information and outputting the results of execution of the machine learning language processing model **111** and the radiotherapy plan optimizer **162.** The electronic platform may include graphical user interfaces (GUI) displayed on each of the end-user devices **120,** the medical device **150,** and/or the medical device computer **152.** An example of the electronic platform generated and hosted by the analytics server **110a** may be a web-based application or a website configured to be displayed on different electronic devices, such as mobile devices, tablets, personal computers, and the like.

The platform hosted on the analytics server **110a** or other device of the system **100** includes collaboration software accessible to the user devices **120** of the participating members of a tumor board or RT professionals during the RTTP process. The collaboration software may include any type of software facilitating user-group collaborations, which may include live interaction software (e.g., teleconferencing software) or asynchronous collaborations (e.g., online postings). Non-limiting examples of the collaboration software may include MS Teams^{®}, Skype^{®}, WebEx^{®}, Slacks, and Twilio^{®}, among others. The analytics server **110a** may execute a language model assisted treatment planning application (TPA) that comprises, invokes, executes, and manages the operations of an artificial intelligence agent (e.g., a program or application configured to interface between the machine learning language processing model **111** and other applications hosted by the analytics server **110a**) and the machine learning language processing model **111**, among other functions. The TPA collects and ingests various types of inputs and feeds the inputs into the artificial intelligence (AI) agent and the machine learning language processing model **111.**

In some embodiments, the TPA (including the AI agent and machine learning language processing model **111**) is a software module component (e.g., plug-in) of the collaboration software of the platform of the analytics server **110a**. In some embodiments, the collaboration software makes calls to the TPA software of the analytics server **110a** to provide inputs to, and invoke operations of, the AI agent and machine learning language processing model **111.**

The information displayed by the TPA of the electronic platform can include, for example, input elements to receive data associated with a patient to be treated (e.g., plan objectives) and display results of predictions for AI-generated text for continuing the tumor board discussion or the RTTP planning discussion, as produced by the machine learning language processing model 111, which may include various formats of responsive predicted outputs (e.g., text, images, or videos generated in response to inputs received through the TPA or electronic platform). In some cases, the outputs produced by the machine learning language processing model **111** of the TPA may be fed to the radiotherapy plan optimizer **162** (e.g., a predicted radiotherapy plan). The radiotherapy plan optimizer **162** may generate attributes (e.g., treatment attributes) of a radiotherapy treatment plan based on the patient attributes and/or treatment attributes. The analytics server **110a** may display the results for the members of the tumor board or participants of the RTTP planning discussion at a user device **120** and/or other medical professionals at the medical device **150.**

The analytics server **110a** may be any computing device comprising a processor and non-transitory machine-readable storage capable of executing the various tasks and processes described herein. The analytics server **110a** may employ various processors such as central processing units (CPU) and graphics processing unit (GPU), among others. Non-limiting examples of such computing devices may include workstation computers, laptop computers, server computers, and the like. While the system **100** includes a single analytics server **110a,** the analytics server **110a** may include any number of computing devices operating in a distributed computing environment, such as a cloud environment.

End-user devices **120** of a tumor board, humans or physicians participating in the RTTP process may be any computing device comprising a processor and a non-transitory machine-readable storage medium capable of performing the various tasks and processes described herein. Non-limiting examples of an end-user device **120** may be a workstation computer, laptop computer, tablet computer, and server computer. In operation, various users may use end-user devices **120** to access the GUI operationally managed by the analytics server **110a.** Specifically, the end-user devices **120** may include a clinic computer **120a**, a clinic server **120b,** and medical professional devices **120c,** which may include any electronic devices operated by members of the tumor board, medical professionals, and scientists that access and review various types of patient-related treatment data and RTTPs for the patient, among other types of data and information exchanges. For instance, members of the tumor board or participants involved in the RTTP process may operate the medical professional devices **120c** to review patient-related treatment data to develop consensus of a diagnosis and RTTP for the patient. Even though referred to herein as "end-user" devices, these devices may not always be operated by end-users. For instance, the clinic server **120b** may not be directly used by an end-user. However, the results stored on the clinic server **120b** may be used to populate various GUIs accessed by an end-user via the medical professional device **120c.** Patient-related information generated by the various types of devices of the system **100,** outside the context of tumor board discussions and/or transcripts of instructions given by an oncologist to other RT experts may be stored in the system database **110b.** The stored patient data and/or transcripts may be referenced by the TPA during tumor board discussions or a collaborative RTTP process and/or referenced by the TPA for training the machine learning language processing model **111** or the AI agents.

The medical device **150** may be a radiation therapy machine configured to implement a patient's radiotherapy treatment. In some embodiments, the medical device **150** can be a diagnostic imaging device and/or a treatment delivery device. The medical device **150** may also be in communication with a medical device computer **152** that is configured to display various GUIs discussed herein. For instance, the analytics server **110a** may display the results predicted by the radiotherapy plan optimizer **162** onto the computing devices described herein.

The machine learning language processing model **111** may be stored in the system database **110b.** The machine learning language processing model **111** may be configured or trained to automatically generate text, image, or video responses based on inputs received at a user interface or other types of inputs (e.g., speech captured at a conference room microphone or microphone of an end-user device **120**). The machine learning language processing model **111** can be configured and trained to receive the various inputs from the members of the tumor board or participants in the RTTP process and patient attributes for a patient as input and automatically generate various predicted responsive outputs for continuing the discussion.

The user-provided inputs can include, for example, text inputs entered by members of the tumor board or participants in the RTTP process via user interfaces (e.g., displayed at the end-user devices **120**) or audio signals containing speech audio of the members of the tumor board or participants in the RTTP process captured by microphones of the user devices **120** or conference room. The TPA may include Automated Speech Recognition (ASR) software that converts speech audio to written text. The ASR software comprises a machine-learning architecture trained to detect portions or frames of the audio signal containing the speech audio of a member speaker. The ASR also comprises and applies Natural Language Processing (NLP) layers of the machine-learning architecture that generates text-based output from the portions of the audio signal containing the detected speech audio. The text generated by the ASR may be fed as an input to the machine learning language processing model **111.** The machine learning language processing model **111** is trained to simulate and contextually continue a conversation with the tumor board or participants in the RTTP process, based upon the text of the medical discussion and the patient-related data indicating the patient attributes over time. The conversations by participants in the RTTP process can be treatment plan (e.g., RT treatment plan) creation discussions.

The analytics server **110a** may execute a radiotherapy plan optimizer **162** to generate one or more treatment attributes for an RTTP complying with any radiation therapy plan objectives based on patient attributes of a patient for which the radiotherapy treatment plan is being generated. The radiotherapy plan optimizer **162** can be stored in the database **160.** The radiotherapy plan optimizer **162** can generate the one or more treatment attributes, for example, by iteratively calculating the one or more treatment attributes where, with each iteration, the radiotherapy plan optimizer **162** can revise the one or more treatment attributes of the RTTP in accordance with a cost value. The analytics server **110a** may deploy the radiotherapy plan optimizer **162** to generate an RTTP for a patient based on patient attributes for the patient. The radiotherapy plan optimizer **162** may iteratively calculate one or more treatment attributes of the RTTP. For instance, with each iteration, the radiotherapy plan optimizer **162** may generate a candidate RTTP having various attributes. The radiotherapy plan optimizer **162** may then use one or more loss functions to calculate a cost value for the generated candidate RTTP. The cost value may indicate a likelihood of the candidate RTTP violating a set of rules (e.g., internal or external rules). For instance, the cost value may indicate whether the candidate RTTP violates any of the plan objectives. The radiotherapy plan optimizer **162** may analyze the cost value. If needed (e.g., when the cost value satisfies a threshold), the radiotherapy plan optimizer **162** may revise the candidate RTTP and re-execute its loss function to generate a new cost value. Depending on whether the new cost function is increasing or decreasing, the radiotherapy plan optimizer **162** may revise the candidate RTTP again and recalculate the cost value. The radiotherapy plan optimizer **162** may continue this iterative approach until converging upon an RTTP (or the final RTTP) that has a cost value that satisfies a threshold.

The analytics server **110a** can identify the treatment attributes that the radiotherapy plan optimizer **162** determined have a cost value that satisfies the threshold. The analytics server **110a** can present the treatment attributes as an RTTP at the end-user device **120** being accessed by the user generating the radiotherapy treatment plan. The user can implement the RTTP or the analytics server **110a** or the end-user device **120** can use the RTTP to automatically control the medical device **150** based on attributes of the RTTP to treat the patient.

The systems and methods described herein can apply to human user or tumor board decisions in generating a radiotherapy treatment plan and RT planning activities. For example, a tumor board can be a multi-disciplinary body that includes experts in various fields on oncology. A tumor board meeting or discussion can involve a discussion or decision making that covers determining different treatment modalities (e.g., surgery, chemotherapy, RT, etc.). RT plan creation during the planning process can include the collaboration of an RT oncologist (e.g., a physician) and one or more planners (e.g., an RT physicist and/or a dosimetrist). The planning process can include several stages of interaction and/or decision making such as taking additional images for RTTP purposes, delineating targets and critical organs, determining the fractionation, setting beam geometries, performing the optimization by defining a set of optimization objectives, evaluating the plan dose distribution (possibly comparing it to some alternative plans) and performing machine QA (to ensure that the machine can actually deliver the plan as expected). In either case, the TPA can enable a language learning model to predict the correct decisions by the respective tumor board for treatment or for the RT plan creation process for determining the next state or stage of interaction and/or decision-making.

### Using Large Language Models to Test the Validity of a Task in Treatment Planning

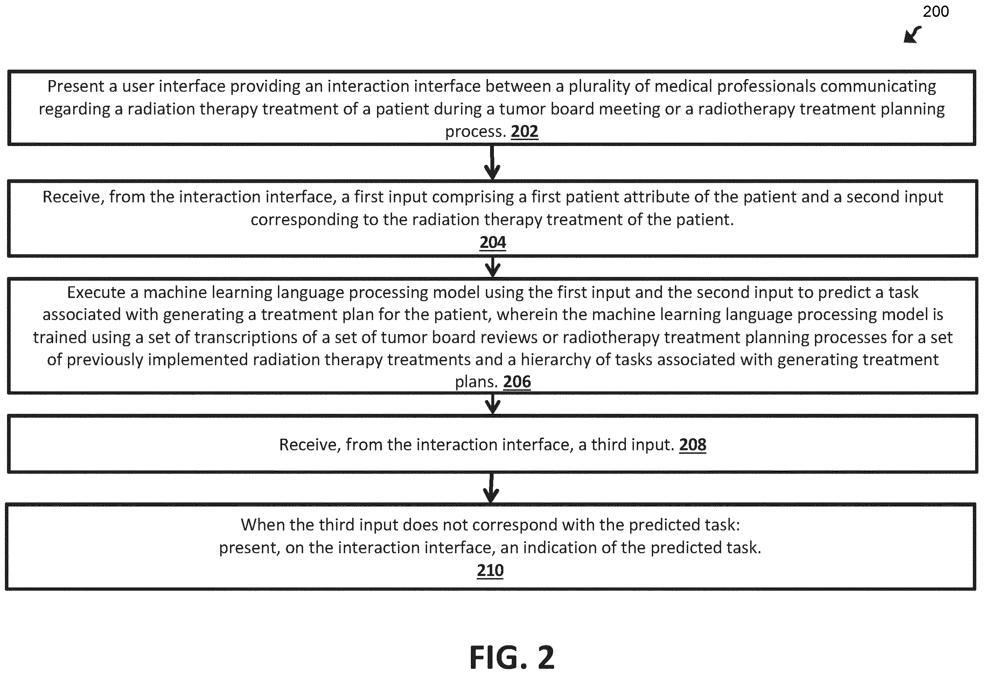

Referring to **FIG. 2****,** a method **200** shows an operational workflow executed in a machine learning language processing plan generation system, in accordance with an embodiment. The method **200** may include steps **202-210.** However, other embodiments may include additional or alternative steps or may omit one or more steps altogether. The method **200** is described as being executed by a server, such as the analytics server described in **FIG. 1****.** However, one or more steps of the method **200** may be executed by any number of computing devices operating in the distributed computing system described in **FIG. 1****.** For instance, one or more computing devices may locally perform part or all of the steps described in **FIG. 2****.**

Using the method **200,** the analytics server can implement a machine learning language processing model to generate a radiotherapy treatment plan or otherwise assist in the RTTP process. To do so, the TPA can host or provide an interaction interface (e.g., conference audiovisual inputs and outputs; a text-based collaboration interface) for capturing inputs from a conference room microphone, or one or more computing device being accessed by one or users (e.g., members of the tumor board determining treatment for a patient during a tumor board review or participants in the RTTP planning process). In some cases, the analytics server hosting the TPA can retrieve patient-related data from a patient database. Through the interaction interface or by capturing various types of user inputs (e.g., microphone capturing voice speech of medical members speaking during tumor board meetings or treatment plan creation discussions), the users can input patient attributes of a patient or any other data, or the TPA may query the patient-related data from the patient database. The analytics server can detect when a task or a decision regarding a task is input into the interaction interface. Responsive to doing so, the analytics server can retrieve inputs collected over the course of the tumor board review or treatment plan creation discussion (e.g., RTTP plan creation discussion of an RTTP plan process) for a patient, in some cases with stored attributes of the patient, and provide the retrieved data as input into a machine learning language processing model. The machine learning language processing model can predict a task or decision based on the input. The analytics server can compare the predicted task with the input task or decision that initiated the prediction by the machine learning language processing model. Responsive to detecting that the predicted task does not match the input, the analytics server can display a message indicating the difference. Otherwise, responsive to determining a match, the analytics server can display a message indicating the match or not display any message at all. In this way, the analytics server can use a machine learning language processing model to automatically observe and validate decisions and inputs of a tumor board or participants in the RTTP process during a tumor board review or treatment plan creation discussions to determine treatment of a patient.

At step **202,** the analytics server presents a user interface on a computing device. The analytics server may generate the user interface of the TPA (or collaboration software having the TPA) and transmit the user interface to the computing device over a network. The analytics server may generate and transmit the user interface to the computing device when providing an electronic platform that facilitates tumor board discussions or treatment plan creation discussions regarding treatment considerations and planning (e.g., RTTP) for patients based on inputs from the members of the tumor board or participants in the RTTP process.

The analytics server can generate the user interface to have an interaction interface. The interaction interface can be an interface that enables the communication between a user viewing and providing inputs into the interaction interface and the analytics server or a machine learning language processing model. The machine learning language processing model can be a large language model that has been trained to generate (i) any combination of one or more of: text, image, and/or video responses to (ii) any combination of one or more of: text, image, and/or video input. The machine learning language processing model can be or include a neural network, such as a neural network with a transformer architecture. The interaction interface can enable a form of communication similar to a conversation between two or more humans that can involve back-and-forth exchanges of messages between participants in a tumor board discussion or treatment plan creation discussion. In this case, the participants may be one or more expert-users who are members of the tumor board or participants of the treatment plan creation discussion, such as an oncologist or another clinician, and an AI agent that implements the machine learning language processing model to ingest inputs from the discussion and provide the outputs of the machine learning language processing model to the interaction interface. The analytics server can provide access to the interactive interface to each participant in the tumor board discussion or participants of the treatment plan creation discussion through the TPA to facilitate the discussion.

The interaction interface can simulate an instant messaging application conversation between the experts and the machine learning language processing model. For example, a user can input text into the interaction interface. The interaction interface can be or include a feed (e.g., a user feed). The text can be or include patient attributes for a patient receiving radiotherapy treatment. For example, the user can select a send button to cause the text to be transmitted to the analytics server. The analytics server can input the text including the patient attributes into the machine-learning language processing model and execute the machine-learning language processing model. Based on the input, the language machine learning model may output a response in text or additional types of information, such as media image data containing image scans, charts/graphs, and the like. The analytics server may present the response in the interaction interface, such as below (e.g., directly or sequentially below) the most recent input text by the user. The user can respond to the response from the machine learning language processing model with text following the response and submit the user input response. This process can repeat any number of times to simulate an instant message application conversation.

The interaction interface can cover a portion of the user interface of the collaboration software or TPA or be a widget of the user interface of the collaboration software or TPA. The user interface can include other portions or widgets that offer differing functionality, such as external software tools. While the interaction interface can enable communication between the tumor board participants or the participants in the RTTP process and the AI agent implementing the machine learning language processing model, another portion of the user interface can display, for example, different patient attributes of patients, treatment options mentioned in the tumor board discussion or treatment plan creation discussion, or other types of outputs generated by a machine learning language processing model. For example, the TPA may capture an audio signal in which a participating member mentions a particular patient attribute, treatment option, or other aspect of the patient's treatment planning, and convert the audio signal into a text input for the AI agent. The machine learning language processing model and the AI agent may be trained to, for example, provide additional information about the particular patient attribute, treatment option, or other aspect of the patient's treatment planning mentioned by the participating member. The AI agent may include this additional information as an output to the user interface presenting the ongoing tumor board discussion or treatment plan creation discussion.

The TPA may receive an input indicating an identifier (e.g., a name) of the patient into the user interface or the platform provided by the analytics server. Responsive to receiving the input, the analytics server can retrieve patient data (e.g., one or more patient attributes) regarding the patient from non-transitory memory containing database records of the patient database. Examples of patient attributes the analytics server may retrieve include any combination of one or more of: computed tomography (CT) scans of the patient or a tumor of the patient, images of the patient or a tumor of the patient, previously collected patient attributes of the patient, such as data collected from previous health tests, among others. The analytics server can present the retrieved patient data on the user interface in another widget or a portion of the user interface separate or adjacent to the interaction interface. In some cases, the portion or widget of the user interface including the retrieved patient data can be separated from the interaction interface on the user interface by a line (e.g., a vertical or horizontal line going across the width or length of the user interface (e.g., along the x-axis or the y-axis) of the user interface).

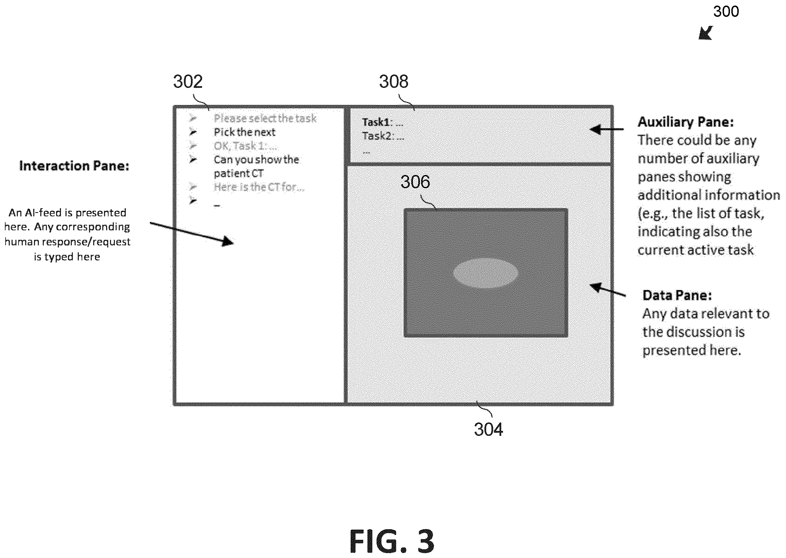

In a non-limiting example, referring now to **FIG. 3****,** the analytics server can generate a user interface **300** to include an interaction interface **302** and a patient data interface **304** of a platform hosting collaboration software and a TPA for contributing to tumor board discussions or treatment plan creation discussions. The interaction interface **302** can be a portion of the user interface **300** that users (e.g., members of a tumor board participating in the discussion) can operate or access to interact with a machine learning language processing model. The user or the TPA can input text, images, and/or video into the interaction interface **302** and receive responses from the machine learning language processing model in the interaction interface **302.** The patient data interface **304** can be an area of the user interface **300** through which the analytics server (e.g., AI agent and machine learning language processing model) can present retrieved patient data **306** for a patient. The patient data interface **304** can be configured to present any type of data, such as text, images, or videos. The analytics server can generate the user interface **300** and transmit the user interface to a computing device accessed by users, such as to physicians and/or members of the tumor board or participants of the RTTP process.

The user interface can include an auxiliary interface **308.** The analytics server can include the auxiliary interface **308** on the user interface **300** to illustrate additional information relevant to the conversation on the interaction interface **302** and for the retrieved data on the patient data interface **304** to the user using the user interface **300.** The auxiliary interface **308** can include a list of tasks, such as the current task. The tasks can indicate tasks for the user to complete by communicating with the machine learning language processing model. For example, the user can input an option to select a task to generate a radiotherapy treatment plan for patient A. Responsive to the input, the machine learning language processing model can identify the input and use the input to determine a response to collected patient data regarding patient A. Another example of a task is to show a CT scan for a patient in the patient data interface **304.** Responsive to an input identifying such a task, the machine learning language processing model can identify the input, and the analytics server can retrieve the CT scan for the patient and display the patient scan on the patient data interface **304.**

Referring again to **FIG. 2****,** at step **204,** the analytics server may receive, from the interaction interface, a first input comprising a first patient attribute of the patient and a second input corresponding to the radiation therapy treatment of the patient. The analytics server can receive the first input from the interaction interface of the user interface. The first input can include a first patient attribute of a patient. The first input can include the first patient attribute and any number of other patient attributes. The first patient attribute and the other patient attributes can be any type of patient attribute, such as, for example, any combination of one or more of: height, gender, weight, treatment options for the patient, treatment attributes (e.g., gantry movements, gantry positions, etc.), treatment objectives, attributes regarding a tumor (e.g., size or shape), images of the patient or tumor, tumor stage, the primary site of treatment, endpoints, whether the tumor has been extended, body mass index, blood pressure, medical history (e.g., previous medical treatments received by the patient, etc.). A user can provide the first input and select a send or submit button on the user interface, for example. The analytics server can receive the first input from the computing device presenting the user interface.

In one example, a user (e.g., a member of the tumor board during the tumor board review or participants involved in a treatment plan creation discussion) can provide the first input by typing the first patient attribute and any other patient attributes of the first input into the interaction interface. For example, the user can type the current height, age, weight, and blood pressure into the interaction interface. Typing the patient attributes into the interaction interface can cause text to appear on the interaction interface. The user can select a submit button responsive to typing the first input into the interaction interface to submit the first input to the analytics server.

In another example, the user can provide the first input by selecting the first attribute and any other patient attributes from patient data that is presented on the user interface. For example, the user can select an image of a tumor of the patient and/or one or more other patient attributes from the patient data. The user can select an option to move the selected patient attributes into the interaction interface or otherwise drag the selected patient attributes into the interaction interface. Upon moving the selected patient attributes into the interaction interface, the user can select an option to submit or send the selected patient attributes to the analytics server. In some cases, the user can select an option to submit or send the selected patient attributes without moving the selected patient attributes to the interaction interface.

In some cases, the user can provide the first input by typing the one or more patient attributes into the interaction interface and selecting one or more patient attributes from the patient data for the patient displayed on the user interface. The user can type one or more patient attributes into the interaction interface, and select one or more patient attributes from the patient data, and select a submit button to transmit the first input to the analytics server. The first input can include any patient attributes for the patient that are input over the course of the tumor board review or treatment plan creation discussion by any number of the participating members of the tumor board or treatment plan creation discussion.

Referring again to **FIG. 3**, in a non-limiting example, the user can provide the first input into the interaction interface **302.** The user can do so, for example, by typing the first patient attribute and any number of other patient attributes into the interaction interface **302.** In some cases, in addition to or instead of typing the first patient attribute into the interaction interface **302,** the user can select the first patient attribute and/or any number of other patient attributes from the patient data interface **304.** The user can select a submit button on the user interface **300** or otherwise submit the patient attributes by selecting an input/output button to transmit the first patient attribute and any other patient attributes to the analytics server.

The second input can correspond with radiation therapy treatment (e.g., the radiation treatment for which the tumor board or participants of the treatment plan creation discussion is planning). The second input can include different treatment attributes for the radiation therapy treatment, such as any combination of one or more of: radiation dosage, areas being treated, the type of the treatment, etc. The analytics server can receive the second input in the same manner as the first input (e.g., from inputs provided by tumor board members or participants of the treatment plan creation discussion at the interaction interface).

Referring again to **FIG. 2**, at step **206**, the analytics server can execute the machine learning language processing model using the first input and the second input to predict a task associated with generated a treatment plan (e.g., an RTTP) for the patient. The task can be a decision or action regarding RTTP to treat the patient. The task can be a task of a hierarchy of tasks associated with generating a radiotherapy treatment plan. For example, the current task being discussed for treating the patient and/or that is currently being implemented to treat the patient is organs-at-risk (OAR) segmentation. A hierarchy of tasks may be or include, in any order or in a specific order and among other tasks, in some cases, objective template selection, optimization, and radiation dose calculation. Each task may correspond to a different treatment attribute or involve a different decision involved in performing the task. The machine learning language processing model may predict a task by predicting one or more decisions and/or treatment attributes associated with performing the task (e.g., predicting text that indicates the one or more decisions and/or treatment attributes). An example of such a decision can include a dose distribution or other pre-defined numerical data (e.g., organ masks). Other examples include selecting treatment templates or other aspects involved in treating the patient.

The machine learning language processing model can be trained to predict the task associated with generating a treatment plan for the patient. The machine learning language processing model can be trained on a set of transcriptions (sometimes referred to as "discussion logs") of a set of tumor board review discussions (e.g., over the course of a meeting) or transcriptions of a set of instructions given by an RT oncologist to other RT experts, stored in one or more databases, for a set of previously implemented RTTPs for other patients. For example, the analytics server can use language processing techniques or machine learning techniques to identify patient attributes, treatment attributes, tasks, and/or decisions for treatment of a patient from a transcription. The analytics server can generate a prompt from the identified patient attributes and treatment attributes and label the prompt with the decisions for treatment. The analytics server can input the labeled prompt into the machine learning language processing model. The machine learning language processing model may output a predicted task. The analytics server can adjust the internal weights and/or parameters of the machine learning language processing model using a loss function and backpropagation techniques based on a difference between the predicted task and the label of the prompt. The analytics server can train the machine learning language processing model in this way over time for any number of transcripts of tumor board review discussions or treatment plan creation discussions.

The analytics server can generate a prompt from the first and/or second input. The prompt can be a feature vector configured to be input into the machine learning language processing model. The analytics server can generate the prompt by configuring the values of the first and/or second input into a specific format. The analytics server can configure the values according to a template that indicates locations to insert specific types of data (e.g., types of patient or treatment attributes of the first input and/or second input), in some cases. The analytics server can insert the prompt generated from the first and/or second input into the machine learning language processing model.

The analytics server can execute the language processing model using the prompt generated from the first and/or second input as an input. The machine learning language processing model can generate a response including a predicted task based on the execution. The response can be or include text. The text can be a string of characters or words predicting a task associated with an RTTP for the patient. The task can include one or more decisions and/or treatment attributes for the patient. One example of a task is a timeline of radiation therapy treatment of the patient. Other examples of tasks can include any combination of one or more of: a decision to perform radiotherapy treatment, field-geometry settings for a radiotherapy machine for treatment of the patient, a prescription for treatment, objectives to use for creating the radiotherapy treatment plan, a treatment protocol selection, using breath holds for treatment, immobilization of the patient, using volumetric arc therapy (VMAT) for treatment, using intensity-modulated radiation therapy (IMRT) for treatment, etc. The machine learning language processing model can generate the response by implementing learned weights and/or parameters on the data of the input prompt generated from the first and/or second inputs.

In some cases, the analytics server can include patient data that was stored in memory in the prompt input to the machine learning language processing model with the first input and/or the second input. For example, the analytics server can receive an input identifier (e.g., a name) of the patient from the computing device. The analytics server can query the memory of the analytics server based on the identifier to retrieve patient data regarding the patient from the memory. The retrieved patient data can include one or more patient attributes of the patient. The analytics server can additionally, in some cases, retrieve treatment protocols that are available to use to treat the patient. The analytics server can identify the one or more patient attributes and/or the treatment protocols retrieved from memory and input the one or more patient attributes and/or the treatment protocols into the machine learning language processing model instead of or in addition to the first input received from the user interface. The machine learning language processing model can determine the response based on the first input (e.g., a first patient attribute and any other patient attributes), the second input, and/or the retrieved patient data.

For example, the analytics server can collect inputs from different tumor board members or human users, over the course of the tumor board review or participants of a treatment plan creation discussion. The analytics server can also collect patient attributes and/or treatment attributes of the patient and store the collected attributes in memory (e.g., prior to or during the discussion of the tumor board review or the treatment plan creation discussion). The analytics server can retrieve the collected attributes (e.g., by querying memory using an identifier, such as a name or identification number, for the patient) and generate a prompt from the collected attributes. The analytics server can generate the prompt according to a defined template that indicates how to format the prompt and/or where to include different types of attributes. The analytics server can input the generated prompt into the machine learning language processing model and execute the machine learning language processing model. Based on the execution, the machine learning language processing model can generate or predict a task associated with generating a treatment plan for the patient.

The analytics server can generate or train the machine learning language processing model. The analytics server can generate or train the machine learning language processing model using supervised learning, unsupervised learning, or semi-supervised learning techniques. For example, the analytics server can train the machine learning language processing model using a labeled training data set. The labeled training data set may include different sentences or paragraphs of text of transcriptions of historical tumor board reviews or transcriptions of sets of instructions given by one RT expert to another RT expert during treatment plan creation discussions. The sentences or paragraphs of text may correspond to the radiotherapy treatment of patients, for example, because the sentences or paragraphs can include values of one or more patient attributes, and may include specific keywords that correspond to radiotherapy treatment (e.g., radiotherapy, radiation, radiation therapy, oncology, tumor, radiation oncology, linear accelerator, radiation dose, external beam radiation), among others. In some cases, the labeled training data set can include stored patient data that corresponds to the patients that were subject to the historical tumor board reviews or treatment plan creation discussions. The labeled training data set can include annotations indicating the rationale behind certain decisions or tasks determined by the tumor board during the tumor board reviews or participants of treatment plan creation discussions. The labeled training data set can additionally or instead include labels indicating the correct responses to the different sentences or text. The analytics server can automatically label the training data set, or a human reviewer can label the training data set. Such text can be fed (e.g., by the analytics server) into the machine learning language processing model for training.

The analytics server can train the machine learning language processing model using backpropagation techniques. For example, the analytics server can insert different entries (e.g., prompts, such as sentences or text) in the training data set into the machine learning language processing model and execute the machine learning language processing model for each entry. In executing the model for an entry, the machine learning language processing model can generate or output a word or a sequence of words based on the words, images, and/or videos in the entry. The analytics server can determine a difference between the output of the machine learning language processing model and the label for the entry according to a loss function. The analytics server can use backpropagation techniques based on the difference to adjust the parameters and/or weights of the machine learning language processing model. The analytics server can train the machine learning language processing model in this manner over time with different labeled entries. The analytics server can train the machine learning language processing model until the machine learning language processing model is accurate to an accuracy threshold, at which point the analytics server can deploy the machine learning language processing model for use to collect patient attributes through the interaction interface.

During training, the analytics server may iteratively execute the machine learning language processing model to generate new predicted text, images, and/or videos based on the training dataset (e.g., for each entry of text, images, and/or videos). If the predicted results do not match the real outcome, the analytics server can continue the training unless and until the computer-generated recommendation satisfies one or more accuracy thresholds and/or is within an acceptable range. For instance, the analytics server may segment the training dataset into three groups (i.e., training, validation, and testing). The analytics server may train the machine learning language processing model based on the first group (training). The analytics server may then execute the (at least partially) trained machine learning language processing model to predict results for the second group of data (validation). The analytics server then verifies whether the prediction is correct. Using the above-described method, the analytics server may evaluate whether the machine learning language processing model is properly trained. The analytics server may continuously train and improve the machine learning language processing model using this method. The analytics server may then gauge the machine learning language processing model's accuracy (e.g., the area under the curve, precision, and recall) using the remaining data points within the training dataset, e.g. the third group of data (testing).

In one example, after deploying the machine learning language processing model, the analytics server can receive inputs of a tumor board review or a treatment plan creation discussion regarding a patient. The analytics server can receive a first input and a second input over the course of the tumor board review or treatment plan creation discussion by receiving one or more patient attributes for the patient, one or more treatment attributes regarding historical treatments performed on the patient, and/or one or more treatment attributes of the treatment being planned. The analytics server can input the first input and the second input, in some cases with stored patient attributes or treatment attributes of the patient, into the machine learning language processing model as a prompt. The analytics server can execute the machine learning language processing model. Based on the execution, the analytics server can predict a task associated with generating a treatment plan for the patient (e.g., a task of such a treatment plan).

At step **208**, the analytics server can receive, from the interaction interface, a third input. The third input can be or include a decision or task of an RTTP for the patient. The analytics server can receive the third input from the interaction interface accessed by a member of the tumor board discussing treatment for the patient in a tumor board review or participants of the treatment plan creation discussion.

In some cases, the analytics server can perform the step **206** based on the third input. For example, the analytics server can receive the third input from an interaction interface being accessed by a board member of the tumor board review or participant in the treatment plan creation discussion at an end user device. Responsive to receiving the third input, the analytics server can determine whether the third input is a "decision," such as a decision regarding treatment of the patient corresponding to the tumor board review or treatment plan creation discussion. The analytics server can make the determination, for example, by executing a machine learning model (e.g., a neural network, a support vector machine, a random forest, etc.) trained to analyze inputs (e.g., text inputs) to identify inputs that indicate a decision regarding treatment of a patient. In one example, the analytics server can compare the third input to text in a database to determine if the input corresponds to a decision. The analytics server can analyze inputs from interaction interfaces of members of the tumor board over the course of the discussion between the members of the tumor board regarding treatment of the patient or participants of the treatment plan creation discussion. The analytics server can identify the third input responsive to determining the third input corresponds to a decision (e.g., a decision regarding a task of treatment of the patient) by at least one member of the tumor board or participant of the treatment plan creation discussion. The analytics server can perform the step 206 responsive to receiving and identifying the third input, in some embodiments.

At step **210**, the analytics server presents the predicted task. The analytics server can present the predicted task at one or more of the interaction interfaces being accessed by members of the tumor board or by participants of the treatment plan creation discussion. For example, the analytics server can transmit the predicted task (e.g., as text) to the end user devices being accessed by the members of the tumor board performing the tumor board review to determine an RTTP for the patient or by participants of the treatment plan creation discussion to determine next stages in generating the RTTP. The end user devices can receive the predicted task and update the interaction interface (e.g., the feed of the interaction interface) with the text of the predicted task. The members of the board or treatment plan creation discussion can see the predicted task and determine next steps regarding treatment (e.g., determine whether to implement the predicted task to treat the patient).

In some cases, the analytics server can compare the predicted task generated by the machine learning language processing model with the third input. In doing so, the analytics server can determine if a condition is satisfied. One example of such a condition may be determining whether the predicted task is an exact match with the third input (e.g., if the predicted task and the third input have the exact same text). In another example, the analytics server can determine whether the predicted task matches the third input within a threshold or tolerance (e.g., if the predicted task and the third input have a number of matching text characters or a number of matching words). In some cases, the analytics server can use an edit distance formula or function to perform this determination.

In another example, the analytics server can determine semantic attributes of the predicted task and/or the third input. The semantic attribute may indicate a meaning or intent of the predicted task and/or the third input. The analytics server can determine the semantic attributes, for example, by providing the predicted task and/or the third input into a machine learning model (e.g., a neural network, a support vector machine, a random forest, etc.) trained to generate semantic attributes for text or other types of inputs. The analytics server can execute the machine learning model using the predicted task and/or third input as input to generate a semantic attribute for the predicted task and/or the third input. The analytics server can compare the semantic attribute generated from the predicted task with the third input, or the semantic attribute of the predicted task with the semantic attribute of the third input, to determine if there is a match. In doing so, the analytics server can determine if there is an exact match or an approximate match, as described above.

The analytics server can update the interaction interfaces being accessed by the members of the tumor board or participants of the treatment plan creation discussion based on the comparison of the predicted task with the third input (e.g., based on the comparison of the semantic attributes of the predicted task and the third attribute). For example, responsive to determining there is a match, the analytics server can present an indication of the match at the interaction interfaces, thus validating the decision of the third input. In some cases, the analytics server may operate in the background and not update the interaction interfaces responsive to determining a match to reduce the amount of content being presented at the interaction interface. However, responsive to determining the predicted task does not match the third input, the analytics server can present the predicted task at the interaction interface, thus indicating to the members of the tumor board or the participants of the treatment plan creation discussion that their decision may not be correct.

In some cases, the analytics server can update the interaction interface (e.g., the interaction interface displayed on the end user devices being accessed by the members of the tumor board or by the participants of the treatment plan creation discussion) with information regarding the predicted task, such as why the machine learning language processing model generated the predicted task or the patient attribute that caused the machine learning language learning model to generate the predicted task. For example, subsequent to determining the predicted task does not match the third input, the analytics server may generate a prompt requesting (e.g., in text) information about the prediction by the machine learning language processing model. The analytics server can execute the machine learning language processing model using the prompt as input. The machine learning language processing model may determine that the prompt is a continuation of the previous prompt based on the context of the prompt and the training of the machine learning language processing model. Responsive to doing so, the machine learning language processing model can output a response indicating any patient attributes or treatment attributes of the patient that caused the machine learning language processing model to generate the predicted task. In some cases, the analytics server can include the third input in the prompt to the machine learning language processing model. In such cases, the machine learning language processing model can output a response indicating any patient attributes that caused the machine learning language processing model to generate a different predicted task than the third input. The analytics server can present the output by the machine learning language processing model at the interaction interfaces being accessed by the members of the tumor board or by the participants of the treatment plan creation discussion.

A member of the tumor board or a participant of the treatment plan creation discussion can indicate an acceptance or a rejection of the predicted task. The member of the tumor board or the participant of the treatment plan creation discussion may do so, for example, by providing an input into the interaction interface indicating that the predicted task is accepted or rejected. An acceptance may mean that the tumor board or the participants of the treatment plan creation discussion determined to use the predicted task to treat the patient or generate the RTTP; while a rejection may mean that the tumor board or the participants of the treatment plan creation discussion determined not to use the predicted task to treat the patient or generate the RTTP. The analytics server can receive the input and execute a machine learning model (e.g., the machine learning language processing model or a different machine learning model) to determine whether the predicted task was accepted or rejected. The analytics server can store an indication of the acceptance or rejection in memory, in some cases with the data (e.g., the first input, the second input, the retrieved patient information, and/or the third input) that resulted in the predicted task and the acceptance or rejection.

The analytics server may use the stored indication, e.g. of the acceptance or rejection of the predicted task, and the data that resulted in the indication, to train the machine learning language processing model. For example, the analytics server can use the stored indication as a label and the data that caused the stored indication to be generated as training data. The analytics server can input the training data into the machine learning language processing system and use backpropagation techniques and a loss function to adjust the weights and/or parameters of the machine learning language processing model. The analytics server may train the machine learning language processing model in this way over time to continually improve the accuracy of the machine learning language processing model.

In some cases, the analytics server can generate (e.g., automatically generate) a radiotherapy treatment plan based on the inputs from members of the tumor board or the participants of the treatment plan creation discussion and/or the outputs from the machine learning language processing model. For example, the analytics server can transmit the selected task (e.g., the predicted task or the task that a member chose to use instead of the selected task), the first input, and/or the second input to a radiation therapy plan optimizer. The analytics server can do so as an instruction to generate a radiotherapy treatment plan for the patient. The radiotherapy plan optimizer can be a computer model (e.g., an optimization computer model) that is configured to generate one or more treatment attributes for a radiotherapy treatment plan that comply with the radiation therapy plan objectives for a patient (e.g., objectives in patient attributes of the patient) based on patient attributes of the patient for which the radiotherapy treatment plan is being generated. The radiotherapy plan optimizer can generate the one or more treatment attributes, for example, by iteratively calculating the one or more treatment attributes where, with each iteration, the radiotherapy plan optimizer revises the one or more attributes of the radiotherapy treatment plan in accordance with a cost value. The radiotherapy plan optimizer can receive the first input, the second input, and/or the predicted task or selected task. Based on the received data (e.g., based on the patient attributes and/or treatment attributes of the received data), the radiotherapy plan optimizer can generate or determine one or more attributes (e.g., radiation dose amounts or other information, field geometry settings, arc settings, treatment frequency, type of treatment, radiation parameters, etc.) of a radiotherapy treatment plan for the patient to generate a radiotherapy treatment plan.

The radiotherapy plan optimizer can receive the data with the instructions from the analytics server. A computer (e.g., the analytics server or another computer) storing the radiotherapy plan optimizer can execute the radiotherapy plan optimizer using the first input, the second input, and/or the response to the predicted task as input. The radiotherapy plan optimizer can output one or more treatment attributes of a radiotherapy treatment plan for the patient based on the patient's attributes. The radiotherapy plan optimizer can transmit the one or more treatment attributes to the analytics server.

The analytics server can receive the one or more treatment attributes. The analytics server can present the radiotherapy treatment plan (e.g., the one or more treatment attributes of the radiotherapy treatment plan) on the user interface. The analytics server can present the radiotherapy treatment plan on the interaction interface of the user interface or on another portion of the user interface. In some embodiments, the analytics server can transmit the one or more treatment attributes to a radiotherapy treatment machine to use to provide treatment to the patient. The radiotherapy treatment machine can operate (e.g., automatically operate) based on the one or more treatment attributes.

In some cases, the user at the user interface may adjust the radiotherapy treatment plan. For example, a member of the tumor board or a participant of the treatment plan creation discussion viewing the radiotherapy treatment plan at the interaction interface can provide an input (e.g., a text input or a selection of a button) at the interaction interface that the radiotherapy treatment plan is incorrect. The analytics server can receive the input indicating the radiotherapy treatment plan is incorrect. Responsive to the input indicating the radiotherapy treatment plan is incorrect, the analytics server can execute the machine learning language processing model using the input or an indication of the input that the radiotherapy treatment plan is incorrect to generate a second response requesting one or more patient attributes regarding the patient. The analytics server can present the second response requesting the one or more patient attributes of the patient at the interaction interface. The analytics server may then receive (further) patient attributes. The analytics server can transmit the first input, the second input, the response to the predicted task, and any further received patient attributes to the radiotherapy plan optimizer. The radiotherapy plan optimizer can generate a second radiotherapy treatment plan for the patient based on the received data. The radiotherapy plan optimizer can transmit the second radiotherapy treatment plan to the analytics server. The analytics server can present the second radiotherapy treatment plan at the user interface for viewing and/or approval by a user (e.g., a member of the tumor board or a participant of the treatment plan creation discussion) and/or transmit the second radiotherapy treatment plan to the radiotherapy machine to trigger the radiotherapy machine to operate according to the second radiotherapy treatment plan.

In some cases, the analytics server can identify a value for a predicted task. The value may be a numerical value for a treatment attribute of the predicted task. For instance, the value can be a radiation dosage, field-geometry settings for a radiation therapy machine, etc. The machine learning language processing model may generate a predicted task output indicating that the next step in treating the patient is to use a radiation therapy treatment plan. Responsive to doing so, the analytics server can execute an analytical protocol (e.g., the radiotherapy plan optimizer or any other type of model configured to evaluate different patient attributes of the patient) to output values for the treatment. The analytics server can present the values at the interaction interface being accessed by one or more members of the tumor board or participants of the treatment plan creation discussion for review to accept, reject, or refine, as described herein.

In some cases, the analytics server can present a hyperlink on the interaction interface. The analytics server can present the hyperlink in the same message or a subsequent message to the message containing the predicted task. The hyperlink can be configured to direct the interaction interface to third-party data associated with the predicted task. The analytics server can select the hyperlink from based on a mapping between the predicted task and the hyperlink in memory, for example. The third-party data can be information describing the predicted task, including a description of the predicted task, risks associated with the predicted task, the benefits of the predicted task, etc. The third-party data can be stored in a remote database hosted by a remote computer. One of the members of the tumor board or one of the participants of the treatment plan creation discussion viewing the interaction interface can select the hyperlink to cause the analytics server to retrieve the third-party data from the remote computer for display to the member that selected the hyperlink and/or any number of the members of the tumor board or participants of the treatment plan creation discussion. The analytics server can display the third-party data on the interaction interface. The members or participants can view the third-party data and use the data to determine whether to accept or reject the predicted task as described herein.

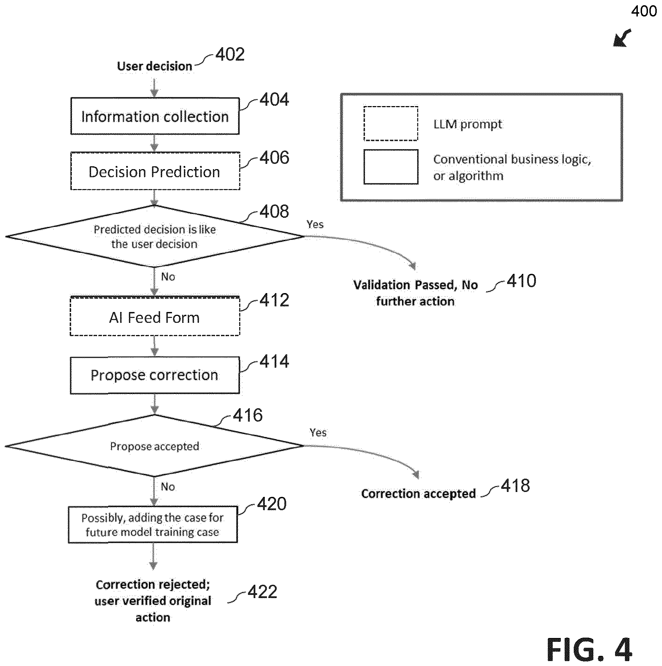

**FIG. 4** illustrates a process flow diagram executed in a machine learning language processing plan generation system, according to an embodiment. The method **400** may include steps **402-422.** However, other embodiments may include additional or alternative steps or may omit one or more steps altogether. One or more of the steps **402-422** can be a step of the method **200** or part of one of the steps of the method **200.** The method **400** is described as being executed by a server, such as the analytics server described in **FIG. 1****.** However, one or more steps of the method **400** may be executed by any number of computing devices operating in the distributed computing system described in **FIG. 1****.** For instance, one or more computing devices may locally perform some or all of the steps described in **FIG. 4****.** The analytics server can perform the steps of the method **400** during a tumor board review meeting or treatment plan creation discussion to correct or validate any proposed actions the tumor board members or participants of the treatment plan creation discussion propose when determining the best course of treatment for a patient or the next stage in the treatment planning process, for example. The steps **402**, **404**, **408**, **410**, and **414-422** can be performed using an LLM prompt (e.g., an input into a machine learning language processing model). Steps **406** and **412** can be performed using a business logic or algorithm.

At step **402**, a user can provide an input into an interaction interface generated by the analytics server. The user can be a member of a tumor board or participant in a treatment plan creation discussion. The user can provide the input during a tumor board review (e.g., a meeting by a tumor board to discuss treatment of a patient or treatment plan creation discussion to discuss stages of treatment for an RTTP for the patient). The analytics server can determine whether the input is a 'decision-making' input. The input can be considered to be decision-making when the input indicates a decision regarding a task to treat a patient or for planning to treat a patient. Examples of decision-making inputs may be any template selection in automated planning, including any user-given choices (e.g., any filling to any formula, including numerical values), any instruction to do additional planning (where one can first assume that there is a decision whether to do additional planning), and/or choices for determining which kind of additional planning to perform. Non-decision-making inputs may correspond to inputs that are not related to treating a patient, such as saving current work, adjusting a visualization, changing an organ label, etc. The analytics server can determine the input is decision-making by comparing the input to data in a database that includes mappings to decision-making inputs, for example. In another example, the analytics server can parse the input using natural language processing techniques to determine an intent or context of the input and determining if the intent or context relates to a task or decision to treat a patient (e.g., by querying a database with the intent or context). In another example, the analytics server can use machine learning techniques to determine whether the input is related to a task or decision to treat a patient. The analytics server can perform any process to determine whether an input relates to treating a patient. The analytics server can receive such inputs over time, or over the course of the tumor board review or the treatment plan creation discussion, and determine whether the inputs are decision-making or not. The analytics server can continue processing inputs in this manner until determining an input is decision-making. As described herein, decisions can be tasks or tasks can be decisions.

At step **404**, the analytics server collects up-stream information that may affect the decision. Such information can be, for example, background patient information (e.g., statements of a radiologist, a report from a previous patient examination, discussions (e.g., previous inputs) in the tumor board meeting or the treatment plan creation discussion, etc.), or a previous decision path for the particular patient. The analytics server can collect and store the information in a database over time, such as before performing the step **402.** The analytics server can retrieve the information for the patient responsive to determining a user provided a decision-making input during the tumor board review or the treatment plan creation discussion. The analytics server can retrieve the information by querying the database using an identifier of the patient. In some cases, the decision is not included in the collected information, but is stored separately. The analytics server can preprocess the data of the latest decision into a serialized format (e.g., the decision can be presented as a vector of tokens, such as coordinates, a selected protocol identification, etc.).

The analytics server can use the retrieved collected information to generate a prompt (e.g., an LLM prompt). For example, the analytics server can generate a machine learning language processing model prompt from the patient attributes of the patient information, historical treatments of similar patients, previous inputs by other individuals participating in the tumor board review or the treatment plan creation discussion, etc. The analytics server can generate the prompt using a template that specifies where to place different types of data. The analytics server may or may not include the most recent input in the prompt. The template may be configured in a manner that causes the machine learning language processing model to predict or generate a "predicted decision." The predicted decision can be a predicted task for a next step in treatment of the patient. In some cases, the analytics server can use a one-shot or few-shot approach and include an example with the input patient attributes to guide the machine learning language processing model to generate a predicted decision. The analytics server can input the prompt into the machine learning language processing model and execute the machine learning language processing model. In response, the machine learning language processing model can output a predicted decision (e.g., a predicted act) regarding treating the patient.

In some cases, instead of initially executing the machine learning language processing model to generate a predicted decision, the analytics server can execute the machine learning language processing model or a different machine learning language processing model to generate a summary or a shorthand version of the information. The analytics server can do so, for example, by including instructions to generate the summary in the input prompt and executing the machine learning language processing model. The analytics server can format the summary into a new prompt (e.g., include the summary with text requesting a predicted decision) and execute the machine learning language processing model with the prompt including the summary. Doing so may be beneficial, for example, when performing a few-shot approach and the machine learning language processing model has a maximum length (e.g., a maximum token length) that limits the size of the allowed input. The summary may have fewer tokens, and therefore enable the few-shot approach. In some cases, the analytics server may generate the summary first responsive to determining the size of the current input prompt is too large for the machine learning language processing model to receive as input.

In some cases, the analytics server can generate multiple predicted decisions. The analytics server may do so by executing the machine learning language processing model multiple times. The analytics server can add varying noise between executions (e.g., in the prompt or by requesting the machine learning language processing model to vary or increase variations in the results). In some cases, the machine learning language processing model may have a high temperature value, which may cause the machine learning language processing model to inherently output different predicted decisions between executions with the same inputs.

The analytics server can determine or evaluate whether the predicted decision differs from the user decision (e.g., the decision that triggered the analytics server to perform the step 404). The analytics server can do so in a few manners, which may depend on the type of the decision. For example, for a single step decision (e.g., a single task or step of treatment), the analytics server can compare the predicted decision with the input decision and determine the two do not differ if there is an exact match, or if the decisions match within a tolerance. For a continuous or multi-step decision (e.g., multiple acts, multiple steps of treatment, multiple treatment attributes, etc.), the analytics server can determine the decisions do not differ if there is an exact match or if there are less than a predetermined number of violations or differences in sub-decisions of the two decisions. The analytics server can determine the decisions do not match, otherwise. In cases in which the analytics server generates multiple predicted decisions, the analytics server may determine a match with the input decision responsive to determining at least one of the multiple predicted decisions match the input decision.

Responsive to determining the predicted decision and the input decision match, at step 408, the analytics server can validate the decision at step 410. In doing so, the analytics server can generate a response (e.g., a text response) indicating the match or that the input decision is correct and transmit or present the response at an interaction interface to the individuals participating in the board review. The analytics server may store an indication of the match or the decision in memory.

However, responsive to determining the predicted decision and the input decision do not match, at step **408,** the analytics server can generate a prompt requesting a message to indicate why the machine learning language processing model generated the predicted decision. The prompt can request the information that caused the machine learning language processing model to generate the predicted decision. The analytics server can execute the machine learning language processing model with the prompt as input. The machine learning language processing model may output identifications of the information (e.g., the patient attributes or other information) that caused the predicted decision.

At step **412**, the analytics server can display the output identifications to the individuals of the tumor board review or the treatment plan creation discussion at the interaction interface. The analytics server can display the output identifications with a message requesting whether to accept or disregard the predicted decision. One of the individuals of the tumor board review or the treatment plan creation discussion can provide an input indicating to accept or disregard the predicted decision. Responsive to receiving an input at step **416** indicating that the predicted decision is accepted, at step **418** the analytics server can store an indication that the predicted decision was accepted.

However, responsive to receiving an input indicating that the predicted decision was not accepted and the input decision would be used for treatment instead, at step **416,** the analytics server can store an indication that the predicted decision was rejected. The analytics server may use the rejection to train **420** the machine learning language processing model, such as by using the rejection as a label with the inputs that resulted in the rejection and by modifying the weights and/or parameters of the machine learning language processing model according to a loss function. In some cases, the analytics server may generate a message requesting why the user rejected the predicted decision and present the message at the interaction interface. The analytics server may store any response that the user provides at the interaction interface. At step **422,** the analytics server can generate and present a message verifying that the predicted decision was rejected at the interaction interface. The acknowledgment may aid in continuing the conversation with any users or members of the tumor board or the treatment plan creation discussion.

In some cases, before deploying the method **400** for predicting decisions to impact treatment, or for specific decisions, in some cases, the analytics server can perform the method **400** 'silently' e.g. without user awareness. For example, the analytics server may receive the inputs from the individuals of a tumor board during a tumor board review or participants of a treatment plan creation discussion over time. The analytics server can perform the method **400** to generate predicted decisions but may not display the predicted decisions at any interaction interfaces being accessed by the individuals of the tumor board or participants of a treatment plan creation discussion. The analytics server can use machine learning techniques (e.g., the machine learning language processing model) to determine the decision that the tumor board or participants of the treatment plan creation discussion settled on using and compare the determined decision with the predicted decision to determine if the two decisions match. In some cases, the analytics server can determine the decision-making inputs that can trigger the method **400** from the decisions that the analytics server determined the tumor board or participants of the treatment plan creation discussion settled on. The analytics server may store a record of matches and/or non-matches and train the machine learning language processing model according to the matches or non-matches. The analytics server may deploy the machine learning language processing model responsive to determining the machine learning language processing model is accurate to a threshold.

Advantageously, by implementing the systems and methods described herein, a computer can find errors in treatment that are difficult to detect. The computer can do so in a more accurate, fast, and efficient manner than other plan validation methods that may focus on quantitative analysis of plan quality, which are based on numerical similarity indexes, or that recognize whether the created plan does not conform with a previous plan. A machine learning language processing model's unique ability to understand natural language based-information and correlate user actions as well as give a natural-language reasoning why the user action was challenged can enable a computer executing such a machine learning language processing model to detect mistakes where some information is overlooked or where the decisions made in different stages of the planning are atypical. These advantages may be compounded when the machine learning language processing is specifically trained to predict decisions for treatment or when single or few-shot prompting techniques are used.

### Using Large Language Models to Predict next User Interactions in Automated Planning Pipelines

The RT treatment planning process can take a relatively long time. One factor increasing the treatment planning time is that the planning contains multiple phases including contribution from many different experts. Planning automatization has improved the situation to some degree when it comes to the need of experts to interact with the algorithms used in various places, but experts typically still need to be involved at some level in the process. For example, many aspects of field geometry generation can be (and have been) automated (such as placing iso-center), but there might be a need for a user to still adjust the result the automation produces. At the same time, automated tasks still take a moderate amount of time, making it inefficient for the experts. For example, experts may still need to wait for certain automated portions of a task to be performed, until the experts can interact directly when the time for interaction arises. The waiting period may cause a hold up because the experts may not be able to work on other tasks while waiting for the automated portions of the tasks to be completed.

An attempt to remove the "human-in-the-loop" problem is to use conventional algorithms or standard supervised machine learning models to make treatment decisions. However, doing so still often involves a human expert (e.g., an oncologist) to review any decisions made by the conventional algorithms or machine learning models. Such methods may involve an iterative approach in which, if the machine learning language processing properly predicts a decision, the planning process can automatically advance to the next stage. However, if the machine learning language processing does not properly predict a decision, algorithms or models may predict another decision and the human expert would have to review the new decision. This process may repeat until the algorithm or model predicts a decision the human expert approves.

A computer can overcome the aforementioned technical problems by using a machine learning language processing model. For example, the computer can be configured to use the machine learning language processing model to predict different tasks of a radiotherapy treatment plan. The different tasks can be or include tasks of a workflow (e.g., a workflow of a radiotherapy treatment plan). In doing so, the machine learning language processing model can recursively determine tasks in sequential order. For instance, starting at an initial act, such as OAR segmentation, the computer can execute the machine learning language processing model to predict a next act, such as objective template selection and/or one or more values of objective template selection. The computer can execute the machine learning language processing model based on any predictions or inputs from the initial task with any other relevant data, such as collected patient attributes or treatment attributes of the patient being treated. The computer can then again execute the machine learning language processing model to predict a third act, such as optimization. The computer can do so based on any predicted values or other predictions from the first and/or second stages of the radiotherapy treatment plan, with any other patient attributes or treatment attributes for the patient. The computer can repeat this process until getting to a final stage, such as dose calculation, or any other stage. Accordingly, the computer can use the machine learning language processing model to automatically generate a workflow of a radiotherapy treatment plan without human intervention. The computer can display the final workflow or any output values from the workflow at a user interface (e.g., an interaction interface) automatically or responsive to receipt of a request.

In some cases, the computer can generate one or more branches from the predicted workflow. The branches can be alternative workflows that begin at one of the predicted tasks of the workflow. The computer can generate the branches at predicted tasks at which a decision or deviation may need to be made. Such predicted tasks can be an "interactive task." An example of an interactive task is objective template selection. For example, when the machine learning language processing model predicts different tasks, the computer can identify the tasks. The computer can compare the individual tasks to stored tasks in a database to determine whether the tasks are interactive tasks. Responsive to determining a predicted task is an interactive task, the computer can execute the machine learning language processing model multiple times (e.g., a predefined number of times) using the same inputs or using varied inputs to generate different predictions to predict different predicted tasks that follow the interactive task. The number of branches that the computer generates can differ based on the interactive task. The computer can determine the number of branches to generate based on data for the interactive task that is stored in the database. The computer can then continue the process of recursively generating tasks for each branch, generating multiple branches for each interactive task, until reaching the final task (e.g., the final stage) for each branch. The computer can store the different workflows in a database for retrieval and display, such as in response to a request. Accordingly, the computer can generate a dynamic web of treatment workflows without human intervention and without waiting for any inputs from different members of the team treating the patient that is typically required to generate the workflow.

The computer can update the workflow over time to display the different outputs of the workflows based on the tasks that a user implements or selects to implement. For example, a member of a tumor board or a participant of a treatment plan creation discussion may select (e.g., via the interactive interface) to implement a predicted task at one of the interactive tasks for which the computer used the machine learning language processing model to generate multiple branches. Based on the selection, the computer can identify the branch of predicted tasks that corresponds with (e.g., that was generated based on) the selected predicted task. The computer can display the branch or the next predicted task of the branch at the interactive interface and/or any outputs of the predicted tasks of the branch. In doing so, the computer can select a single branch at each interactive task of the selected branch and display the data or values of each of the selected branches. Accordingly, the computer can display multiple predicted tasks at any point in the workflow to show a recommended workflow of treatment for a patient without waiting for inputs from individuals that are involved in the treatment.

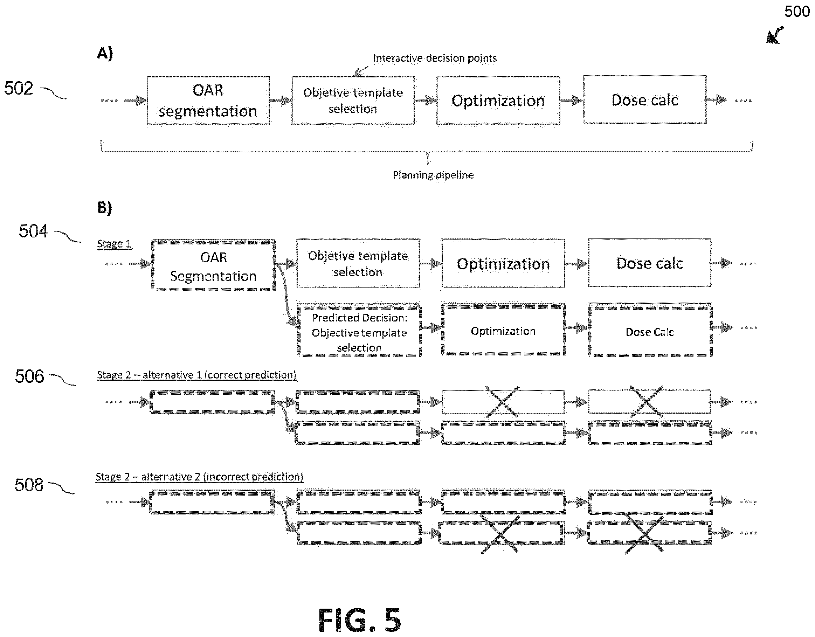

For example, **FIG. 5** illustrates a dataflow **500** amongst components of a system for hosting tumor board or RTTP planning process collaboration and radiotherapy treatment plan generation, according to an embodiment. The dataflow **500** can include a sequence of determining a radiotherapy treatment plan using a large language model using the systems and method described herein. The dataflow **500** is described as being implemented using a server, such as the analytics server described in **FIG. 1****.** However, the dataflow **500** may be executed by any number of computing devices operating in the distributed computing system described in **FIG. 1****.** For instance, one or more computing devices may locally perform some or all of the steps or processes described in **FIG. 5****.** The dataflow **500** can be implemented during a tumor board review meeting or treatment plan creation discussion to correct or validate any proposed actions of different workflows the tumor board members or participants of the treatment plan creation discussion propose when determining the best course of treatment for a patient, for example. The dataflow **500** can be performed using a machine learning language processing model (e.g., a large language model).

The dataflow **500** can include an example workflow **502** of a planning pipeline. The workflow **502** can include different tasks of a workflow for determining treatment attributes of a radiotherapy treatment plan or stages of developing a treatment plan. The tasks can occur in sequential order. The analytics server can generate the workflow **502** by recursively executing a machine learning language processing model as described above. As illustrated in the example workflow **502,** the objective template selection may be an interactive task of the example workflow **502.** The analytics server can identify the objective template selection task as an interactive act. In response to doing so, the analytics server can generate multiple alternative workflows **504** and **506** using the machine learning language processing model. The analytics server can select which workflow **504** or **506** to use based on inputs by a user at a user interface.

For example, in a stage one **508,** the analytics server can automatically generate the workflow **502** until identifying the interactive task of objective template selection. Responsive to doing so, instead of stopping the process and waiting for a user interaction, the computer can execute the machine learning language processing model to predict alternative workflows **504** and **506.** A user may select or input a predicted task at the interactive task of one of the workflows **504** and **506.** Responsive to selecting one of the predicted tasks (e.g., selecting the workflow **504**), the computer can validate the predicted task. The analytics server can do so by executing the machine learning language processing model using the selected predicted task as input. The machine learning language processing model may generate a message asking the user to confirm the selected predicted task. Upon receiving an input confirming the selection, the analytics server may identify (e.g., without executing the machine learning language processing model again) the branch of the workflow that was generated based on the selected predicted task to be the main flow. The analytics server can display any outputs of the main flow at the interaction interface. The analytics server can discard any alternative branches of the workflow, such as the workflow **506**, increasing memory space for any future predictions for the same patient's treatment or for a different patient's treatment.

The dataflow **500** can be implemented to aid a tumor board or participants of a treatment plan creation discussion in generating a radiotherapy treatment plan for treating a patient. For example, the analytics server can receive inputs from members of a tumor board during a tumor board review of a patient or participants of a treatment plan creation discussion for a patient, as described above with respect to **FIGS. 1-4**. Based on the inputs, the analytics server can generate a workflow with multiple branches at interactive tasks of the workflow. The analytics server can display a predicted task of the workflow at an interactive task of one or more of the members of the tumor board or participants of the treatment plan creation discussion responsive to determining the members or participants provided an input for the same task and the analytics server determining the input does not match the predicted act. The member of the tumor board or participant of the treatment plan creation discussion can determine to use the predicted task or the input task and provide an input into the interactive interface indicating the decision. Responsive to receiving an input indicating not use to the displayed predicted act, the computer may identify a branch that the machine learning language processing model generated that includes the input task instead of the displayed predicted act. The computer can display any results or data of the new branch at the interactive interface or otherwise continue to monitor the inputs of the tumor board or participants of the treatment plan creation discussion against the newly selected branch of the workflow. The analytics server can train the machine learning language processing model based on differences or similarities between the predicted tasks and the implemented tasks over time to improve the accuracy of the machine learning language processing model or otherwise account for any changes in treatment philosophies or methodologies.

In a non-limiting example, during a tumor board review or treatment plan creation discussion for determining a treatment of a patient or a stage of determining a treatment plan for a patient, a group of board members or participants of a treatment plan creation discussion, in some cases with an oncologist treating the patient, can discuss different options of tasks for treating the patient. In doing so, the participants in the tumor board review or participants in the treatment plan creation discussion may provide inputs into an interaction interface provided by an analytics server. The analytics server can detect when the board members provide an input indicating a task, such as a type of treatment, a dosage of radiation to use for treatment, or the next stage in generating a treatment plan for the patient. Responsive to detecting the input indicating the task, the analytics server can execute a machine language processing model using other inputs that the analytics server collected during the communication (e.g., such as patient attributes, treatment attributes of prior treatments of the patient, treatment attributes of the treatment being prescribed, etc.) as input. In some cases, the analytics server can input previously collected patient and/or treatment attributes regarding the patient with data collected during the tumor board review or treatment plan creation discussion. The machine learning language processing model can output a predicted task based on the inputs. The analytics server can compare the predicted task with the input task. Responsive to determining the input and the predicted task match, the analytics server can update the interaction interface to indicate the match.

However, responsive to determining the input and the predicted task do not match, the analytics server can execute the machine learning language processing model again with instructions to indicate any data that may have caused the machine learning language processing model to generate an alternative to the input task. The machine learning language processing model can output indications of the data that caused the different prediction in a message. The analytics server can display the message at the interaction interface being accessed by one or more of the individuals involved in the tumor board review or the treatment plan creation discussion. At least one of the individuals may provide an input indicating whether to implement the predicted task or the previously input task. The analytics server can store a record of the input to use for further predictions of tasks for treating the patient during the tumor board review or the treatment plan creation discussion and/or to train the machine learning language processing model. In some cases, the analytics server may have used the machine learning language processing model to generate multiple workflows. The analytics server can select which workflow to display or use to make further predictions based on the predicted task that an individual participating in the tumor board review or treatment plan creation discussion selects. The analytics server can display any data predicted for the workflow, such as in response to an input at the interaction interface.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of this disclosure or the claims.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc., may be passed, forwarded, or transmitted via any suitable means including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions may be stored as one or more instructions or code on a non-transitory computer-readable or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. It' A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein may be applied to other embodiments without departing from the scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope being indicated by the following claims.

### Clauses

The following numbered clauses are subject-matter of, but not currently claims of, the present EP application. **That is, the following clauses are part of the description, and are not claims.** The Applicant reserves the right to claim this subject-matter in this EP application.
1. A method comprising:
   presenting, by a processor, a user interface providing an interaction interface between a plurality of medical professionals communicating regarding a radiation therapy treatment of a patient during a tumor board meeting or a radiotherapy treatment planning process;
   receiving, by the processor from the interaction interface, a first input comprising a first patient attribute of the patient and a second input corresponding to the radiation therapy treatment of the patient;
   executing, by the processor, a machine learning language processing model using the first input and the second input to predict a task associated with generating a treatment plan for the patient, wherein the machine learning language processing model is trained using a set of transcriptions of a set of tumor board meetings or radiotherapy treatment planning processes for a set of previously implemented radiation therapy treatments and a hierarchy of tasks associated with generating treatment plans;
   receiving, by the processor from the interaction interface, a third input; and
   when the third input does not correspond to the predicted task:
      presenting, by the processor on the interaction interface, an indication of the predicted task.
2. The method of clause 1, further comprising:
   when the third input corresponds with the predicted task:
   executing, by the processor, an analytical protocol to identify a value for the predicted task; and
   presenting, by the processor, the value for the predicted task on the interaction interface.
3. The method of clause 1 or clause 2, wherein the predicted task is associated with a timeline of radiation therapy treatment of the patient.
4. The method of any preceding clause, further comprising:
   transmitting, by the processor, the first input, the second input, and a response to the predicted task to a radiation therapy plan optimizer as an instruction to generate a radiotherapy treatment plan for the patient.
5. The method of any preceding clause, further comprising:
   presenting, by the processor on the interaction interface, a hyperlink configured to direct the interaction interface to third-party data associated with the predicted task.
6. The method of any preceding clause, further comprising:
   receiving, by the processor, a response to presentation of the predicted task; and
   retraining, by the processor, the machine learning language processing model using the response.
7. The method of any preceding clause, further comprising:
   generating, by the processor, an alternative workflow that does not include the predicted task.
8. A system, comprising:
   a server comprising a processor and a non-transitory computer-readable medium containing instructions that when executed by the processor causes the processor to:
   present a user interface providing an interaction interface between a plurality of medical professionals communicating regarding a radiation therapy treatment of a patient during a tumor board meeting or a radiotherapy treatment planning process;
   receive, from the interaction interface, a first input comprising a first patient attribute of the patient and a second input corresponding to the radiation therapy treatment of the patient;
   execute a machine learning language processing model using the first input and the second input to predict a task associated with generating a treatment plan for the patient, wherein the machine learning language processing model is trained using a set of transcriptions of a set of tumor board meetings or radiotherapy treatment planning processes for a set of previously implemented radiation therapy treatments and a hierarchy of tasks associated with generating treatment plans;
   receive, from the interaction interface, a third input; and
   when the third input does not correspond to the predicted task, present, on the interaction interface, an indication of the predicted task.
9. The system of clause 8, wherein the instructions further cause the processor to:
   when the third input corresponds with the predicted task:
   execute an analytical protocol to identify a value for the predicted task; and
   present the value for the predicted task on the interaction interface.
10. The system of clause 8 or clause 9, wherein the predicted task is associated with a timeline of radiation therapy treatment of the patient.
11. The system of any of clause 8 to clause 10, wherein the instructions further cause the processor to:
   transmit the first input, the second input, and a response to the predicted task to a radiation therapy plan optimizer as an instruction to generate a radiotherapy treatment plan for the patient.
12. The system of any of clause 8 to clause 11, wherein the instructions further cause the processor to:
   present, on the interaction interface, a hyperlink configured to direct the interaction interface to third-party data associated with the predicted task.
13. The system of any of clause 8 to clause 12, wherein the instructions further cause the processor to:
   receive a response to presentation of the predicted task; and
   retrain the machine learning language processing model using the response.
14. The system of any of clause 8 to clause 13, wherein the instructions further cause the processor to:
   generate an alternative workflow that does not include the predicted task.
15. A system, comprising:
   a computer configured to display a user interface; and
   a server in communication with the computer, the server configured to:
      present the user interface providing an interaction interface between a plurality of medical professionals communicating regarding a radiation therapy treatment of a patient during a tumor board meeting or a radiotherapy treatment planning process;
      receive, from the interaction interface, a first input comprising a first patient attribute of the patient and a second input corresponding to the radiation therapy treatment of the patient;
      execute a machine learning language processing model using the first input and the second input to predict a task associated with generating a treatment plan for the patient, wherein the machine learning language processing model is trained using a set of transcriptions of a set of tumor board meetings or radiotherapy treatment processes for a set of previously implemented radiation therapy treatments and a hierarchy of tasks associated with generating treatment plans;
      receive, from the interaction interface, a third input; and
      when the third input does not correspond to the predicted task, present, on the interaction interface, an indication of the predicted task.
16. The system of clause 15, wherein the server is further configured to:
   when the third input corresponds with the predicted task:
   execute an analytical protocol to identify a value for the predicted task; and
   present the value for the predicted task on the interaction interface.
17. The system of clause 15 or clause 16, wherein the predicted task is associated with a timeline of radiation therapy treatment of the patient.
18. The system of any of clause 15 to clause 17, wherein the server is further configured to:
   transmit the first input, the second input, and a response to the predicted task to a radiation therapy plan optimizer as an instruction to generate a radiotherapy treatment plan for the patient.
19. The system of any of clause 15 to clause 18, wherein the server is further configured to:
   present, on the interaction interface, a hyperlink configured to direct the interaction interface to third-party data associated with the predicted task.
20. The system of any of clause 15 to clause 20, wherein the server is further configured to:
   receive a response to presentation of the predicted task; and
   retrain the machine learning language processing model using the response.

## Claims

1. A method comprising:
presenting, by a processor, a user interface providing an interaction interface between a plurality of human users such as medical professionals communicating regarding a radiation therapy treatment of a patient during a tumor board meeting or a radiotherapy treatment planning process;
receiving, by the processor from the interaction interface, a first input comprising a first patient attribute of the patient and a second input corresponding to the radiation therapy treatment of the patient;
executing, by the processor, a machine learning language processing model using the first input and the second input to predict a task associated with generating a treatment plan for the patient, wherein the machine learning language processing model is trained using a set of transcriptions of a set of tumor board meetings or radiotherapy treatment planning processes for a set of previously implemented radiation therapy treatments and a hierarchy of tasks associated with generating treatment plans;
receiving, by the processor from the interaction interface, a third input; and
when the third input does not correspond to the predicted task:
presenting, by the processor on the interaction interface, an indication of the predicted task.

2. The method of claim 1, further comprising:
when the third input corresponds with the predicted task:
executing, by the processor, an analytical protocol to identify a value for the predicted task; and
presenting, by the processor, the value for the predicted task on the interaction interface.

3. The method of claim 1 or claim 2, wherein the predicted task is associated with a timeline of radiation therapy treatment of the patient.

4. The method of any preceding claim, further comprising:
transmitting, by the processor, the first input, the second input, and a response to the predicted task to a radiation therapy plan optimizer as an instruction to generate a radiotherapy treatment plan for the patient.

5. The method of any preceding claim, further comprising:
presenting, by the processor on the interaction interface, a hyperlink configured to direct the interaction interface to third-party data associated with the predicted task.

6. The method of any preceding claim, further comprising:
receiving, by the processor, a response to presentation of the predicted task; and
retraining, by the processor, the machine learning language processing model using the response.

7. The method of any preceding claim, further comprising:
generating, by the processor, an alternative workflow that does not include the predicted task.

8. A system, comprising:
a server comprising a processor and a non-transitory computer-readable medium containing instructions that when executed by the processor causes the processor to:
present a user interface providing an interaction interface between a plurality of human users such as medical professionals communicating regarding a radiation therapy treatment of a patient during a tumor board meeting or a radiotherapy treatment planning process;
receive, from the interaction interface, a first input comprising a first patient attribute of the patient and a second input corresponding to the radiation therapy treatment of the patient;
execute a machine learning language processing model using the first input and the second input to predict a task associated with generating a treatment plan for the patient, wherein the machine learning language processing model is trained using a set of transcriptions of a set of tumor board meetings or radiotherapy treatment planning processes for a set of previously implemented radiation therapy treatments and a hierarchy of tasks associated with generating treatment plans;
receive, from the interaction interface, a third input; and
when the third input does not correspond to the predicted task, present, on the interaction interface, an indication of the predicted task.

9. The system of claim 8, wherein the instructions further cause the processor to:
when the third input corresponds with the predicted task:
execute an analytical protocol to identify a value for the predicted task; and
present the value for the predicted task on the interaction interface.

10. The system of claim 8 or claim 9, wherein the predicted task is associated with a timeline of radiation therapy treatment of the patient.

11. The system of any of claim 8 to claim 10, wherein the instructions further cause the processor to:
transmit the first input, the second input, and a response to the predicted task to a radiation therapy plan optimizer as an instruction to generate a radiotherapy treatment plan for the patient.

12. The system of any of claim 8 to claim 11, wherein the instructions further cause the processor to:
present, on the interaction interface, a hyperlink configured to direct the interaction interface to third-party data associated with the predicted task.

13. The system of any of claim 8 to claim 12, wherein the instructions further cause the processor to:
receive a response to presentation of the predicted task; and
retrain the machine learning language processing model using the response.

14. The system of any of claim 8 to claim 13, wherein the instructions further cause the processor to:
generate an alternative workflow that does not include the predicted task.

15. The system of any of claim 8 to claim 14, comprising:
a computer configured to display the user interface; and
the server in communication with the computer.
